# EUROPEAN PATENT SPECIFICATION

(11) **EP 1 345 610 B1**
(45) Date of publication and mention of the grant of the patent: **06.12.2006**
(21) Application number: 01271220.4
(22) Date of filing: 18.12.2001
(51) Int. Cl.: A61K 31/554, A61K 31/198, A61K 31/48, A61P 25/14, A61P 25/16

(54) **Quetiapine for treating of dyskinesia in non-psychotic patients**
Quetiapin zur Behandlung der Dyskinesie in nicht-psychotischen Patienten
QUETIAPINE POUR LE TRAITEMENT DE LA DYSKINESIE DANS DES PATIENTS NON-PSYCHOTIQUE

(30) Priority: 20.12.2000 US 287582 P
(43) Date of publication of application: 24.09.2003
(73) Proprietor: AstraZeneca AB, 151 85 Södertälje (SE)
(72) Inventor: GOLDSTEIN, Jeffrey, Wilmington, DE 19850-5437 (US)
(86) International application number: PCT/SE2001/002820
(87) International publication number: WO 2002/049652

(56) References cited:
- US-A- 4 879 288
- FRANCESCO BIBBIANA ET AL.: '5HT2A antagonist improves levodopa-induced dyskinesias in MPTP lesioned cynomolgus monkeys' NEUROLOGY vol. 56, no. 8 (SUPPL. 3), April 2001, pages A376 - A377, NO. S46.005, XP002950300
- DATABASE BIOSIS [Online] DOC. NO. PREV200200038621 OH J.D. ET AL.: 'Serotonin 5HT2A antagonist attenuates levodopa-induced motor response alterations in rodent and primate parkinsonian models', XP002950201 Retrieved from STN Database accession no. 2002:38621 & ANNUAL MEETING OF THE SOCIETY FOR NEUROSCIENCE vol. 27, no. 2, 10 November 2001 - 15 November 2001, SAN DIEGO, CALIFORNIA, USA, page 2571
- JOSEPH H. FRIEDMAN MD ET AL.: 'Atypical antipsychotics in the treatment of drug-induced psychosis in Parkinson's disease' MOVEMENT DISORDERS vol. 15, no. 2, March 2000, pages 201 - 211, XP002950202
- STEVEN D. TARGUM MD ET AL.: 'Efficacy of quetiapine in Parkinson's patients with psychosis' JOURNAL OF CLINICAL PSYCHOPHARMACOLOGY vol. 20, no. 1, 2000, pages 54 - 60, XP002950203
- HUBERT H. FERNANDEZ MD ET AL.: 'Quetiapine for the treatment of drug-induced psychosis in Parkinson's disease' MOVEMENT DISORDERS vol. 14, no. 3, 1999, pages 484 - 487, XP002950204
- WILLIAM J. WEINER MD ET AL.: 'Quetiapine for L-dopa-induced psychosis in PD' NEUROLOGY vol. 54, no. 7, April 2000, page 1538, XP002950205
- HUBERT H. FERNANDEZ: 'Quetiapine for L-dopa-induced psychosis in PD' NEUROLOGY vol. 55, no. 6, September 2000, page 899, XP002950206
- DATABASE MEDLINE [Online] DOC. NO. 20294278 VESELY C. ET AL.: 'Remission of severe tardive dyskinesia in a schizophrenic patient treated with the atypical antipsychotic substance quetiapine', XP002950207 Retrieved from STN Database accession no. 2000458990 & INTERNATIONAL CLINICAL PHSYHOPHARMACOLOGY vol. 15, no. 1, January 2000, pages 57 - 60
- VESELY C. ET AL: 'Remission of severe tardive dyskinesia in a schizophrenic patient treated with the atypical antipsychotic substance quetiapine' INTERNATIONAL CLINICAL PSYCHOPHARMACOLOGY vol. 15, no. 1, January 2000, LONDON, pages 57 - 60, XP001156058

## Description

This invention relates to a method for treating dyskinesias associated with dopaminergic therapy in non-psychotic patients and in particular to the use of quetiapine in treating such disorders. The invention also relates to a method for treating Parkinson's disease in non-psychotic patients by administration of quetiapine and a dopaminergic agent.

Quetiapine is an atypical antipsychotic agent which has good efficacy and tolerability and which is useful in the treatment of schizophrenia.

Dopaminergic therapy is a typical treatment for alleviating or partially alleviating the symptoms of Parkinson's Disease. Unfortunately, behavioural disorders and psychoses are complications of dopaminergic therapy and are difficult to treat.

Parkinson's Disease is a gradually progressive neurological disorder resulting from the loss of dopamine from that part of the brain responsible for co-ordination of motor movements, i.e. the basal ganglia. The main symptoms of Parkinson's Disease include tremor (shaking of the hands, feet or limbs), rigidity (stiffness, pain and cramps in muscles), bradykinesia (slowness of movement) and posture changes (difficulty in walking, maintaining balance, freezing). The cause of Parkinson's disease has yet to be determined, although it is not a hereditary disorder, and there is at present no cure for Parkinson's disease. The main treatment is drug therapy aimed at replacing the lost dopamine.

Quetiapine has been used in treating psychoses in patients suffering from Parkinson's Disease with no exacerbation of extra-pyramidal symptoms (Matheson and Lamb, Adis, CNS Drugs 2000, 14(2), 157-172). Matheson and Lamb conclude that quetiapine may be an effective alternative treatment to other antipsychotic agents without compromising motor function. Reference may also be made to two papers which describe the possibility of administering atypical antipsychotics as an adjunct to levodopa to treat the psychoses caused by levodopa therapy; Fernandez and Friedman: The role of atypical antipsychotics in the treatment of movement disorders, CNS Drugs11(6):467-483, 1999; and to Friedman and Factor: Atypical antipsychotics in the treatment of drug-induced psychosis in Parkinson's disease, Movement Disorders 15(2):201-211,2000.

We have now found that quetiapine is useful in treating dyskinesias associated with dopaminergic therapy. Dyskinesias are restless, jerky, uncontrollable movements that can result from complications of dopaminergic therapy.

The present invention relates to a method for treating dyskinesias associated with dopaminergic therapy which comprises administering an effective amount of quetiapine or a pharmaceutically acceptable salt thereof to a non-psychotic patient in need thereof.

In another aspect, the present invention relates to quetiapine or a pharmaceutically acceptable salt thereof for use in treating dyskinesias associated with dopaminergic therapy in a non-psychotic patient.

In yet a further aspect, the present invention provides the use of quetiapine or a pharmaceutically acceptable salt thereof in the manufacture of a medicament for treating dyskinesias associated with dopaminergic therapy in a non-psychotic patient.

By treating dyskinesias we include both reduction of dyskinesias and prophylactic treatment to prevent or ameliorate increased dyskinesias due to other therapy, for example dopaminergic therapy.

Conventional treatment of Parkinson's Disease includes treatment with levodopa and with related drugs such as dopaminergic agents. Levodopa and related drugs give rise to levodopa-induced dyskinesia; the familiar motor function problems that are observed in patients suffering from Parkinson's Disease. Treatment with quetiapine will suppress the symptoms of Parkinson's Disease and will attenuate levodopa-induced dyskinetic movements. This allows the dosage of dopaminergic agents, for example levodopa, to be increased without the complicating side-effects normally observed with higher doses.

Therefore, in another aspect the present invention relates to a method for treating dyskinesias in a patient in need thereof which comprises co-administering to said patient a dopaminergic agent in an amount that together with quetiapine or a pharmaceutically acceptable salt thereof is effective to provide greater efficacy than provided by administering said dopaminergic agent alone.

Furthermore, treatment with quetiapine will enable a larger dosage of other drugs used to treat Parkinson's Disease or Parkinsonian symptoms. We further provide a method for treating dyskinesias associated with Parkinson's Disease which comprises co-administering an effective amount of quetiapine or a pharmaceutically acceptable salt thereof to a patient in need thereof together with a treatment for Parkinson's disease whereby the efficacy of the co-administered treatment is greater than that provided by the treatment for Parkinson's Disease alone.

In a further aspect we provide a method for treating Parkinson's Disease in a non-psychotic patient in need thereof which comprises concomitant administration of quetiapine or a pharmaceutically acceptable salt thereof and a dopaminergic agent; in particular where the dose of dopaminergic agent is greater than that administered to said patient in the absence of quetiapine or a pharmaceutically acceptable salt thereof. A particular benefit of such treatment is that the increased dose of dopaminergic agent enables the improved treatment of Parkinson's Disease and its symptoms without increased dyskinetic side-effects. Examples of dopaminergic agents include levodopa, bromocriptine, pergolide and amantadine. Preferably the dopaminergic agent is levodopa. Typically levodopa may be administered with a decarboxylase inhibitor for example carbidopa or benserazide; in particular levodopa and carbidopa may be administered concomitantly.

Quetiapine is 11-(4-[2-(2-hydroxyethoxy)ethyl]-1-piperazinyl)dibenzo[b,f][1.4]-thiazepine. This compound, pharmaceutically acceptable salts thereof and its use in treating schizophrenia are described in granted European Patent No. EP 240,228 and in corresponding patents.

The method of treatment of the present invention may be conducted over a short term (5-6 weeks), medium term (1-6 months) or long term (6 months - 2 years or more) treatment, and is particularly valuable in medium term and long term treatment. In a particular aspect, quetiapine does not exhibit the significant weight gain seen with some other atypical antipsychotics. Thus, it is particularly suitable for longer term treatment.

Quetiapine may be administered as the compound, 11-(4-[2-(2-hydroxyethoxy)ethyl]-1-piperazinyl)-dibenzo[b,f][1.4]thiazepine or may be administered in the form of a pharmaceutically acceptable salt. Examples of suitable salts include, for example, chloride, maleate, fumarate, citrate, phosphate, methane sulphonate and sulphate salts. Preferred salts include fumarates and a particularly preferred salt is the hemi-fumarate.

It is generally preferred that 11-(4-[2-(2-hydroxyethoxy)ethyl]-1-piperazinyl)-dibenzo[b,f] [1.4]thiazepine is administered in the form of a pharmaceutically acceptable salt, and in particular a fumarate (2:1) salt.

In the treatment of the diseases and conditions mentioned above quetiapine or a pharmaceutically acceptable salt may be administered orally or parenterally in a conventional dosage form such as tablets, pills, capsules, injectables or the like. The dosage in mg/kg of body weight of the compound used to treat mammals will vary according to the size of the mammal and particularly with respect to the brain/body weight ratio. In general, a higher mg/kg dosage for a small animal such as a dog will have the same effect as a lower mg/kg dosage in an adult human. A minimum effective dosage for quetiapine or a pharmaceutically acceptable salt thereof will be about 0.5 mg/kg of body weight per day for mammals with a maximum dosage for a small mammal such as a dog, of about 200 mg/kg per day.

For humans, a dosage of about 0.5 to 25mg/kg per day will generally be effective. Typically, a dosage of about 50mg to 1200mg per day will generally be effective. Usually, a dosage of about 25mg to about 1000mg per day will be administered, with a convenient dosage being about 50-750mg per day. In particular, a dosage of about 50-250mg per day may be preferred such as about 75-150mg per day. The dosage can be given once daily or in divided doses, for example, 2 to 4 doses daily. The dose may be conventionally formulated in an oral or parenteral dosage form by compounding 50 to 1200 mg per unit dosage of conventional vehicle, excipient, binder, preservative, stabiliser, flavour or the like as called for by accepted pharmaceutical practice, for example, as described in US Patent 3,755,340.

Quetiapine or a pharmaceutically acceptable salt may be used in pharmaceutical compositions as the sole active ingredient or may be contained in a pharmaceutical composition together with one or more other active ingredients, or it may be co-administered with one or more known drugs.

Dopaminergic agents typically may be administered according to methods known in the art for such agents, in dosage forms, at unit doses and at frequencies as determined by the skilled medical practitioner. For example levodopa typically may be administered orally from one to four times a day with a total daily dosage of 200mg to 1200 mg dependent on the patient's condition, body weight and other factors.

As indicated above, where quetiapine or a pharmaceutically acceptable salt is administered in conjunction with another agent it may be co-administered simultaneously, sequentially or separately with that other agent or agents. Thus, as indicated above, quetiapine or a pharmaceutically acceptable salt may be formulated with the other agent or agents or may be presented as a separate formulation.

Thus, in one aspect of the present invention, there is provided a pharmaceutical composition comprising quetiapine or a pharmaceutically acceptable salt and a dopaminergic agent, for example levodopa, together with a pharmaceutically acceptable diluent or carrier.

In a further aspect there is provided a pharmaceutical composition comprising quetiapine or a pharmaceutically acceptable salt and a dopaminergic agent, for example levodopa, for simultaneous, sequential or separate administration.

In a yet further aspect of the present invention, there is provided a pharmaceutical composition comprising quetiapine or a pharmaceutically acceptable salt and an agent for treating Parkinson's Disease or Parkinsonian symptoms together with a pharmaceutically acceptable diluent or carrier.

In another aspect there is provided a pharmaceutical composition comprising quetiapine or a pharmaceutically acceptable salt and an agent for treating Parkinson's Disease or Parkinsonian symptoms for simultaneous, sequential or separate administration.

The preparation of 11-(4-[2-(2-hydroxyethoxy)ethyl]-1-piperazinyl)-dibenzo[b,f][1.4] thiazepine and its pharmaceutically acceptable salts is described in, for example, granted European Patents Nos. EP 240,218; EP 282,236 and in International Patent Application No. PCT/GB98/02260. This compound is commercially available under the generic name quetiapine fumarate.

The invention will now be illustrated with reference to the following, non-limiting examples in which quetiapine was used as the fumarate (2:1) salt..

### Example 1

The benefit of quetiapine may be demonstrated as follows:

Quetiapine may be administered to cynomolgus monkeys exposed to 1-methyl-4-phenyl-1,2,3,6-tetrahydropyridine (MPTP) and which therefore have MPTP-induced Parkinson's Disease. Such monkeys are described by Blanchet et al., Experimental Neurology, 153, 214-222 (1998).

The monkeys are assessed according to the Laval University Disability Scale for MPTP monkeys with the following motor and behavioural parameters scored: posture (normal, flexed, crouched); mobility (normal, passive); climbing (present, absent); gait (normal, abnormal); tremor (present, absent); holding food (present, absent); vocalising (present, absent); grooming (present, absent); and social interaction (present, absent).

Quetiapine may be administered at various dosage values, either alone or with varying dosages of levodopa, and scoring is carried out at regular intervals.

Quetiapine was administered subcutaneously to 8 cynomolgus monkeys at doses of 2mg/Kg and 4mg/Kg. The monkeys had been treated with a dose of levodopa either sufficient to produce moderate dyskinesias or sufficient to produce maximum dyskinesias. Quetiapine at a dose of 4mg/Kg reduced moderate levodopa-induced dyskinesias by 57% and reduced maximum levodopa-induced dyskinesias by 41%. Quetiapine at a dose of 2mg/Kg was less effective. The levodopa effect on Parkinson's Disease symptoms was unchanged.

Quetiapine can reduce dyskinesia in the cynomolgus monkeys whilst the levodopa response is maintained.

### Example 2

The following illustrates representative pharmaceutical dosage forms containing the compound 11-(4-[2-(2-hydroxyethoxy)ethyl]-1-piperazinyl)-dibenzo[b,f][1,4] thiazepine fumarate (2:1).

| (a) Tablet | mg/tablet |
|---|---|
| Quetiapine fumarate | 50.0 |
| Mannitol, USP | 223.75 |
| Croscarmellose sodium | 6.0 |
| Maize starch | 15.0 |
| Hydroxypropylmethylcellulose (HPMC), | 2.25 |
| Magnesium stearate | 3.0 |

| (b) Capsule | |
|---|---|
| Quetiapine fumarate | 10.0 |
| Mannitol, USP | 488.5 |
| Croscarmellose sodium | 15.0 |
| Magnesium stearate | 1.5 |

The above formulations may be obtained by conventional procedures well known in the pharmaceutical art. The tablets may be enteric coated by conventional means, for example to provide a coating of cellulose acetate phthalate.

A preferred formulation is that available commercially as quetiapine fumarate.

### Example 3

Levodopa is administered orally twice a day at a dosage of up to 2000mg per day concomitantly with quetiapine fumarate (25 mg twice a day) to a patient in need thereof.

## Claims

1. The use of quetiapine or a pharmaceutically acceptable salt thereof in the manufacture of a medicament for treating dyskinesias associated with dopaminergic therapy of non-psychotic patients, **characterized in that** the dopaminergic agent is selected from levodopa, bromocriptine, pergolide and amantadine.

2. The use according to claim 1 wherein the dyskinesias are associated with levodopa therapy.

3. The use according to claim 1 wherein the dopaminergic agent is administered to alleviate symptoms of Parkinson's Disease.

4. The use according to claim 2 wherein the levodopa is administered to alleviate symptoms of Parkinson's Disease.

5. A pharmaceutical composition which comprises a dopaminergic agent and quetiapine or a pharmaceutically acceptable salt thereof, **characterized in that** the dopaminergic agent is selected from levodopa, bromocriptine, pergolide and amantadine.

6. The use according to claim 3 wherein the quetiapine or a pharmaceutically acceptable salt thereof and the dopaminergic agent are administered concomitantly.

7. The use according to claim 6 wherein the dose of dopaminergic agent is greater than that administered to said patient in the absence of quetiapine or a pharmaceutically acceptable salt thereof.

8. The use according to claim 6 or 7 wherein the dopaminergic agent is levodopa.

9. The use according to claim 6 or 7 wherein the dopaminergic agent is levodopa and carbidopa.

## Patentansprüche

1. Verwendung von Quetiapin oder eines pharmazeutisch verträglichen Salzes davon bei der Herstellung eines Medikamentes zur Behandlung von Dyskinesien, die mit einer dopaminergen Therapie nichtpsychotischer Patienten einhergeht, **dadurch gekennzeichnet, dass** das dopaminerge Mittel aus Levodopa, Bromcriptin, Pergolid und Amantadin ausgewählt ist.

2. Verwendung nach Anspruch 1, wobei die Dyskinesien mit einer Levodopa-Therapie einhergehen.

3. Verwendung nach Anspruch 1, wobei das dopaminerge Mittel zur Linderung der Symptome von Parkinson-Krankheit verabreicht wird.

4. Verwendung nach Anspruch 2, wobei das Levodopa zur Linderung der Symptome von Parkinson-Krankheit verabreicht wird.

5. Pharmazeutische Zusammensetzung, umfassend ein dopaminerges Mittel und Quetiapin oder ein pharmazeutisch verträgliches Salz davon, **dadurch gekennzeichnet, dass** das dopaminerge Mittel aus Levodopa, Bromcriptin, Pergolid und Amantadin ausgewählt ist.

6. Verwendung nach Anspruch 3, wobei das Quetiapin oder ein pharmazeutisch verträgliches Salz davon und das dopaminerge Mittel gleichzeitig verabreicht werden.

7. Verwendung nach Anspruch 6, wobei die Dosis des dopaminergen Mittels größer ist als diejenige, die dem Patient bei Fehlen von Quetiapin oder einem pharmazeutisch verträglichen Salz davon verabreicht wird.

8. Verwendung nach Anspruch 6 oder 7, wobei das dopaminerge Mittel Levodopa ist.

9. Verwendung nach Anspruch 6 oder 7, wobei das dopaminerge Mittel Levodopa und Carbidopa ist.

## Revendications

1. Utilisation de la quétiapine ou d'un sel de celle-ci acceptable d'un point de vue pharmaceutique dans la fabrication d'un médicament destiné à traiter des dyskinésies associées à une thérapie dopaminergique de patients non psychotiques, **caractérisée en ce que** l'agent dopaminergique est choisi parmi la lévodopa, la bromocriptine, le pergolide et l'amantadine.

2. Utilisation selon la revendication 1, dans laquelle les dyskinésies sont associées à une thérapie par lévodopa.

3. Utilisation selon la revendication 1, dans laquelle l'agent dopaminergique est administré pour soulager des symptômes de la maladie de Parkinson.

4. Utilisation selon la revendication 2, dans laquelle la lévodopa est administrée pour soulager des symptômes de la maladie de Parkinson.

5. Composition pharmaceutique qui comprend un agent dopaminergique et de la quétiapine ou un sel de celle-ci acceptable d'un point de vue pharmaceutique, **caractérisée en ce que** l'agent dopaminergique est choisi parmi la lévodopa, la bromocriptine, le pergolide et l'amantadine.

6. Utilisation selon la revendication 3, dans laquelle la quétiapine ou un sel de celle-ci acceptable d'un point de vue pharmaceutique et l'agent dopaminergique sont administrés de manière concomitante.

7. Utilisation selon la revendication 6, dans laquelle la dose d'agent dopaminergique est supérieure à celle administrée au dit patient en l'absence de quétiapine ou d'un sel de celle-ci acceptable d'un point de vue pharmaceutique.

8. Utilisation selon la revendication 6 ou 7, dans laquelle l'agent dopaminergique est la lévodopa.

9. Utilisation selon la revendication 6 ou 7, dans laquelle l'agent dopaminergique est la lévodopa et la carbidopa.
